# EUROPEAN PATENT APPLICATION

(11) **EP 2 789 364 A1**
(43) Date of publication of application: **15.10.2014**
(21) Application number: 13162738.2
(22) Date of filing: 08.04.2013
(51) Int. Cl.: A61N 1/05, A61N 1/36

(54) **A lead, especially a lead for neural applications**

(71) Applicant: Sapiens Steering Brain Stimulation B.V., 5656 AE Eindhoven (NL)
(72) Inventor: Ouchouche, Sébastien Jody, 5583 TD Waalre (NL)
(74) Representative: Prinz & Partner mbB

(57) **Abstract**

The present invention relates to a lead (300), especially a lead (300) for neural applications, preferably a lead (300) for a neurostimulation and/or neurorecording system, wherein the lead (300) comprises at least one length adjustment mechanism (400), wherein the length adjustment mechanism (400) is configured such that the length of the lead (300) is adjustable. Furthermore, the present invention relates to a neurostimulation and/or neurorecording system (100), a thin film (301), a fixations means and a locking mechanism.

## Description

The present invention relates to a lead, especially for a lead for neural applications, a neurostimulation and/or neurorecording system, a thin film, a fixations means and a locking mechanism.

Implantable neurostimulation devices have been used for the past ten years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the probes are connected to an implantable current pulse generator.

Currently, systems are under development with more, smaller electrodes in a technology based on thin film manufacturing. These novel systems consist of a lead made from a thin film based on thin film technology, as e.g. described in WO 2010/055453 A1. The thin films are fixed on a carrier material to form a probe. These probes will have multiple electrode areas and will enhance the precision to address the appropriate target in the brain and relax the specification of positioning. Meanwhile, undesired side effects due to undesired stimulation of neighbouring areas can be minimized.

Leads that are based on thin film manufacturing are e.g. described by US 7,941,202 and have been used in research products in animal studies.

In existing systems, the DBS lead has e.g. four 1.5 mm-wide cylindrical electrodes at the distal end spaced by 0.5 mm or 1.5 mm. The diameter of the lead is 1.27 mm and the metal used for the electrodes and the interconnect wires is an alloy of platinum and iridium. The coiled interconnect wires are insulated individually by fluoropolymer coating and protected in an 80A urethane tubing. With such electrode design, the current distribution emanates uniformly around the circumference of the electrode, which leads to stimulation of all areas surrounding the electrode.

The lack of fine spatial control over field distributions implies that stimulation easily spreads into adjacent structures inducing adverse side-effects in about 30% of the patients. To overcome this problem, systems with high density electrode arrays are being developed, hence providing the ability to steer the stimulation field to the appropriate target.

Silicone-based electrode arrays have long been used as high density electrode arrays, such as the Michigan array or the Utah array. However, the mechanical mismatch between the stiff probe and soft biological tissue may cause inflammation at the implant site. The inflammation encourages the formation of glial scar which encapsulates the probe and thus isolates the electrodes.

Therefore, implantable devices with a soft and flexible base structure are needed. Polyimide is traditionally chosen as the mechanical supporting and electrical insulation material for many such implantable electrode designs due to its biocompatibility, lower stiffness and ease of fabrication. However, polyimide suffers from high moisture absorption, which can lead to metal delamination from the polymeric substrate.

To overcome this problem, microelectrode arrays which are mechanically supported and electrically insulated by other flexible materials such as Liquid Crystal Polymer (LCP) or Parylene are currently being developed. Various conductive materials like gold, platinum are used for the electrodes or traces connecting them and interconnecting processes such as ultrasonic bonding, ball bonding etc. are used to connect them to silicone-based shanks or PCBs with signal processing circuits.

One other alternative, is to form an integrated thin film lead having a plurality of electrodes forming a complex electrode geometry, wherein the electronics of the lead are partially integrated into the lead and the thin film, the thin film e.g. providing both flexible microelectrode array and micro fabricated conductors. Once integrated in the flexible lead body of the lead, the electrodes and conductors must survive the implantation procedure as well as exhibit long term reliability.

Furthermore, because of the fact that the depth of implantation varies from one patient to another, the length of the lead cable or the lead connector elements need to be long enough to accommodate for the depth variations. Additional length might be also required for lead stabilization or that components of the system do not interfere. This leads e.g. to a lead length of about 15 cm when typically only about 6-9 cm are required to reach the target area. Additionally, the lead impedance per track is proportional to the lead length.

So, it would be beneficial for the system if the lead length could be variable or significantly shorter.

It is therefore an object of the present invention, to improve a lead, especially for a lead for neural applications, a neurostimulation and/or neurorecording system, a thin film, a fixations means and a locking mechanism, in particular in that the impedance of the lead may be decreased and that the unnecessary parts of the lead which increase the length of the lead can be avoided.

The above object is solved according to the present invention by a lead according to claim 1. Accordingly, a lead is provided, wherein the lead comprises at least one length adjustment mechanism, wherein the length adjustment mechanism is configured such that the length of the lead is adjustable.

By this, the advantage is achieved that the length of the lead may be adjusted e.g. during implantation. So, the really needed length of the lead may be provided. This allows accommodating different sizes of brains, or application of the lead to different depths. Further, a lead can be provided with no exposed part of the lead on the patient's skull. Since the flexible part of the lead is located beneath the skull, no protective means (such as reinforced tubing for instance) are required as well. Additionally, the advantage is achieved that the impedance of the lead may be decreased and that the unnecessary parts of the lead which increase the length of the lead can be avoided.

Especially, the lead may be a lead for neural applications, preferably a lead for a neurostimulation and/or neurorecording system, e.g. a lead for a deep brain stimulation system.

The lead may e.g. comprise at least one thin film, whereby the thin film comprises a proximal end and a distal end, the lead further comprising a plurality of electrodes on the distal end of the thin film.

The thin film may include at least one electrically conductive layer, preferably made of a biocompatible material. The thin film may be assembled to the carrier and further processed to constitute the lead element. The thin film for a lead is preferably formed by a thin film product having a distal end, a cable with metal tracks and a proximal end. The distal end of the thin film may be forming a part of the distal end of the lead or substantially the distal end of the lead.

The distal end of the lead may be the end of the lead, which is in the implanted state of the lead the remote end of the lead with regard to the body surface area. In particular, in case of a lead for brain application, the distal end of the lead is the lower end of the lead, which is remote to the burr-hole of the skull, through which the lead is implanted.

There may be an Active Lead Can element, which may comprise electronic means to address the plurality of electrodes and at least one Active Lead Can connecting means. Further, the Active Lead Can element may be hermetically or substantially hermetically sealed and may comprise electronic means to address the plurality of electrodes on the distal end of the thin film, which is arranged at the distal end and next to the distal tip of the lead. The plurality of electrodes may comprise more than 5-10 electrodes, e.g. 16 or 32 electrodes or in preferred embodiments e.g. 40 electrodes or more. The electrodes may be arranged such that the electrodes are substantially evenly distributed arranged all over the distal end of the lead.

Moreover, it is possible that the length adjustment mechanism comprises at least one tubular section and/or at least one rod-shaped section and/or that the length adjustment mechanism comprises or is a tube and/or a rod. Such a tubular section and/or at least one rod-shaped section provides in particular the advantage that the stability of the lead may be increased and that the length adjustment of the lead may be improved.

Furthermore, it is possible that the least length adjustment mechanism comprises at least one partially spirally wound section, wherein the at least one partially spirally wound section is configured such that the length of the lead is adjustable, especially that the at least one partially spirally wound section is configured such that the length of the lead is adjustable by extending or compressing the one partially spirally wound section. The spirally wound section comprises the advantage that the spirally shaped structures allows an easy length adjustment, in particular by the fact that spirally wound section allows by structure an adjustment of the length of the lead by extending or compressing the one partially spirally wound section. Furthermore and advantageously, such a length adjustment of the lead by extending or compressing the one partially spirally wound section does in particular substantially not affect the stability of the lead.

In particular, it is possible that the spirally wound section forms a spring structure and/or that spirally wound section is connected to the tubular section. Further, it is possible that the lead comprises a thin film and that the thin film is at least partially attached to the length adjustment mechanism. The thin film may provide at least partially the connecting lines from the electronic means of the system to the electrodes of the lead. By attaching the thin film to the length adjustment mechanism the length of the thin film is automatically adjusted to the correct length.

Additionally, it is possible that the lead comprises at least one reinforcement means, which is configured and/or arranged such that the overall structure of the lead is at least partially stabilized and/or reinforced, wherein especially the reinforcement means is at least partially made of a biocompatible and/or biostable polymeric material, wherein further especially the polymeric material is PEEK and/or polyurethane or the like.

Moreover, it is possible that the reinforcement means is connectable and/or insertable and/or attachable with or into the length adjustment mechanism and/or that the length adjustment mechanism is connectable and/or insertable and/or attachable with or into the reinforcement means.

Additionally, it is possible that the reinforcement means and/or the length adjustment mechanism comprises a length indication means and/or an azimuthal orientation means, exemplarily at least one fiducial marker, and/or that the reinforcement means and/or the length adjustment mechanism is configured such that the relative position to at least one element of the length adjustment mechanism is indicated. In particular, a length indication means provided e.g. the advantage that the surgeon during implantation may easily check the correctness of the length and/or the azimuthal orientation. The fiducial marker may be a marker which is directly visible and/or visible with imaging technologies such as X-Ray, computer tomography (CT), magnetic resonance imaging (MRI) or the like.

Furthermore, it is possible that the length adjustment mechanism is at least partially directly and/or indirectly covered by a protective coating, wherein especially the protective coating is a biocompatible and/or biostable protective coating. By this coating, the advantage may be achieved that a smooth and lubricous surface of the lead may be at least partially provided. Such a surface may improve and allow a smoother implantation of the lead. Further, the coating allows e.g. clear separation of electronic parts from the surrounding tissue and increase the biocompatibility of the lead.

Moreover, it is possible that the lead comprises a locking mechanism which is configured such that the position of the at least one partially spirally wound section and at least one fixation means is lockable such that the length of the lead is fixable after the length adjustment.

Furthermore, it is possible that the least length adjustment mechanism is at least partially made of a biocompatible and/or biostable material, wherein the biocompatible and/or biostable material is exemplarily a biocompatible and/or biostable polymer such as silicone, PEEK, polyurethane or the like and/or a biocompatible and/or biostable metal and/or metal alloy, and where further exemplarily the at least one tubular section and/or at least one rod-shaped section and/or the tube and/or the rod and/or the at least one partially spirally wound section is at least partially made of a biocompatible and/or biostable material, wherein the biocompatible and/or biostable material is exemplarily a biocompatible and/or biostable polymer such as silicone, PEEK, polyurethane or the like and/or a biocompatible and/or biostable metal and/or metal alloy.

Further, the present invention relates to a neurostimulation and/or neurorecording system with the features of claim 12. Accordingly, a neurostimulation and/or neurorecording system is provided, especially a deep brain stimulation (DBS) system, comprising at least one lead according to any claims 1 to 11.

Additionally, the present invention relates to a thin film with the features of claim 13. Accordingly, a thin film comprises the thin film features according to any of the claims 1 to 11.

Moreover, the present invention relates to a fixation means with the features of claim 14. Accordingly, a fixation means comprises the fixation means features according to any of the claims 1 to 11.

Furthermore, the present invention relates to a locking mechanism with the features of claim 15. Accordingly, a locking mechanism comprises the locking mechanism features according to any of the claims 1 to 11.

The locking mechanism may be a snap-fit locking mechanism. Also, the locking mechanism may comprise a snap-fit locking mechanism.

Moreover, a method for adjusting the length of a lead is disclosed. Accordingly, the length of the lead is adjusted, e.g. during implantation of the lead. In particular, the lead may comprise at least one length adjustment mechanism, wherein the length adjustment mechanism is configured such that the length of the lead is adjustable.

After the adjustment of the length and/or the azimuthal orientation of the lead the length and/or the azimuthal orientation may be fixated and locked.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe neurostimulation system for deep brain stimulation (DBS) and its components;
- Fig. 3:: a schematical drawing of a probe system according to the present invention;
- Fig. 4:: a schematical drawing of a DBS lead connected to an Active Lead Can according to the present invention;
- Fig. 5:: a schematical drawing of an embodiment of a length adjustment means;
- Fig. 6:: a schematical drawing of a length adjustment mechanism with a tubular and a spirally wound section;
- Fig. 7:: a schematical drawing of the length adjustment mechanism according to Figure 6 and together with the thin film;
- Fig. 8:: a schematical drawing of the length adjustment mechanism according to Figure 7 and together with the depth tubing;
- Fig. 9:: a schematical drawing of the depth tubing; and
- Fig. 10:: a schematical drawing of the length adjustment mechanism according to Figure 8 covered by a protective coating.

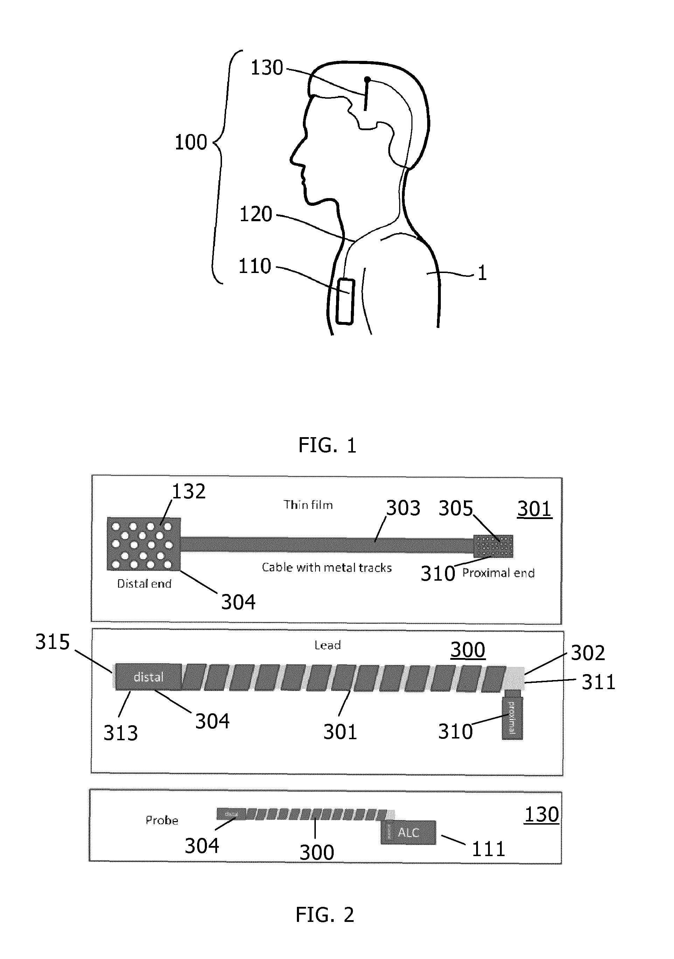

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a deep brain stimulation probe 130 that comprises a DBS lead 300 and an Active Lead Can element 111 comprising electronic means to address electrodes 132 on the distal end 304 of the thin film 301, which is arranged at the distal end 313 and next to the distal tip 315 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 arranged at the proximal end 311 of the lead 300 is electrically connected to the Active Lead Can element 111. The Active Lead Can element 111 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises metal lines (not shown) to connect each distal electrodes 132 to a designated proximal contact 305.

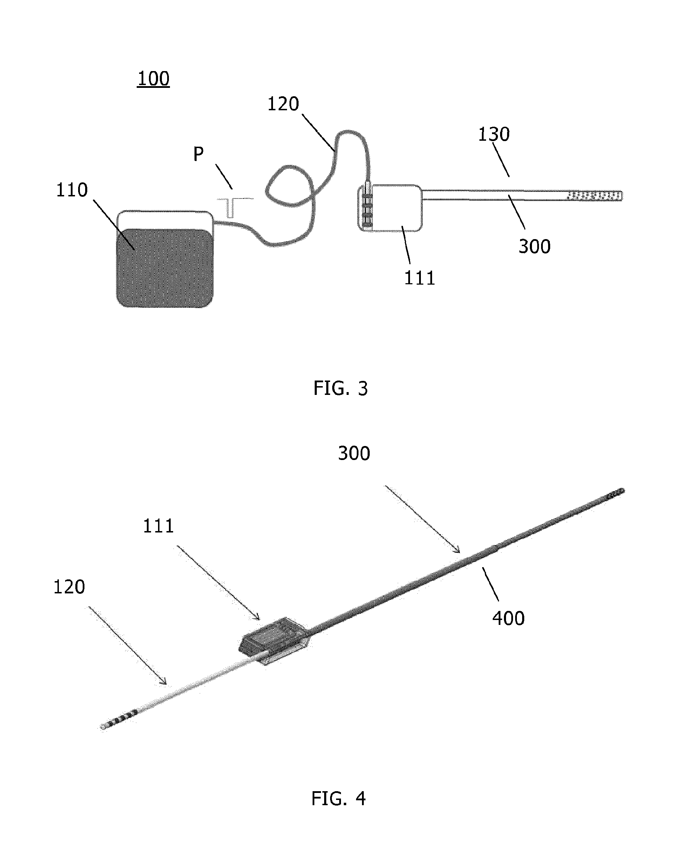

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the Active Lead Can 111. The controller 110 can be an implantable pulse generator (IPG) 110.

Figure 4 shows a schematical drawing of a DBS lead 300 connected to an Active Lead Can element 111. The lead 300 comprises a length adjustment mechanism 400, which is configured such that the length of the lead 300 is adjustable.

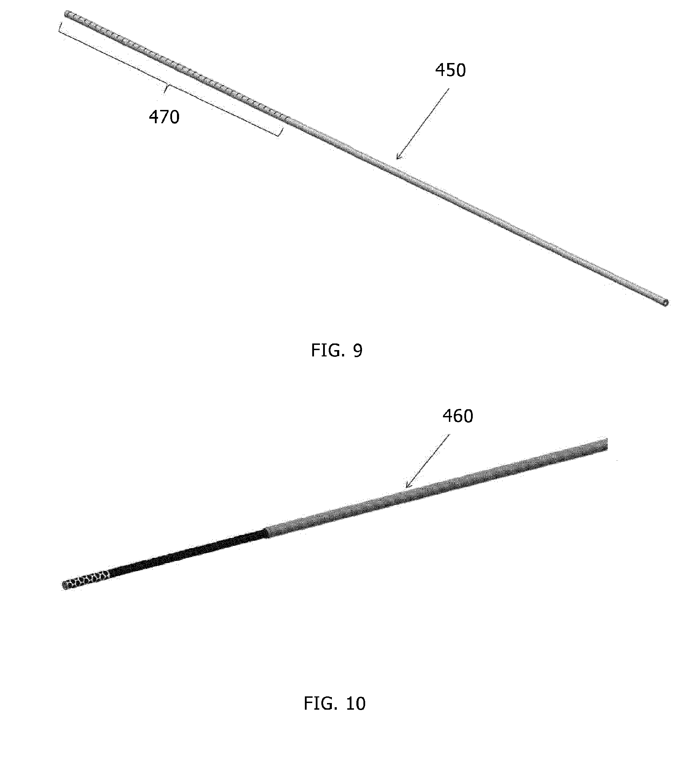

So, the length of the lead 300 is easily adjustable prior to and preferably also during implantation. Further, the implantation of the lead 300 does not require bending of the lead 300 and no additional stabilization is required. Additionally, no protective tubing is required to protect the lead 300 from crushing forces or to firmly secure the lead in the cranium. A reliable and accurate determination of azimuthal orientation of the lead is possible, as described hereinafter by means of fiducial marker 470 (see Figure 9). The diameter of the lead 300 can be minimized as no protective means are required for the exposed part of the lead 300. It is possible that the lead is easily implantable with existing implantation equipment, in particular stereotactic frames and microdrives,

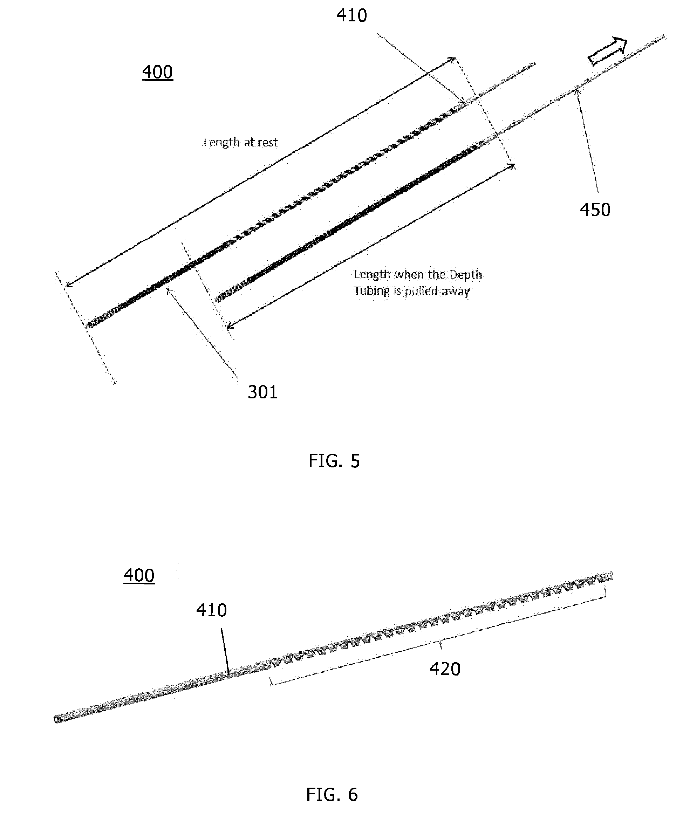

The details of the structure of a possible embodiment of the length adjustment mechanism 400 are shown in Figure 5.

The lead 300 comprises a thin film 301 and the thin film 301 is at least partially attached to the length adjustment mechanism 400. So, the length of the lead is made adjustable by having the thin film 301 bonded to a tube 410, referred as the core 410, which has a middle portion acting as a spring structure 420 (see Figure 6). The core 410 is distally attached to a tube referred as the depth tubing 450 or also reinforcement means 450, which is longer than the core 410 and extends proximal to the Active Lead Can 111 (not shown, see e.g. Figure 4).

Lowering or pulling of the depth tubing 450 respectively elongates or shortens the length of the lead 300 by stretching or compressing the spring structure 420 of the core 410. Once the length of the lead 300 is set to its targeted length, a clamping mechanism or locking mechanism (not shown) locks the depth tubing 450 in place, hence preventing the lead 300 from getting back to its initial length at rest.

As can be further seen in Figure 6, the length adjustment mechanism 400 comprises at least one tubular section 410 and/or at least one rod-shaped section and/or that the length adjustment mechanism 400 comprises or is a tube and/or a rod.

The length adjustment mechanism 400 comprises at least one partially spirally wound section 420, wherein the at least one partially spirally wound section is configured such that the length of the lead 300 (see e.g. Figure 4) is adjustable, especially that the at least one partially spirally wound section 420 is configured such that the length of the lead 300 is adjustable by extending or compressing the one partially spirally wound section 420.

The core 410 of the lead 300 is a tube that has a major portion of its surface area removed (for instance through laser machining) to form a spring structure 420. The core 410 is preferably made of biocompatible and/or biostable polymer such as silicone, PEEK, polyurethane etc.

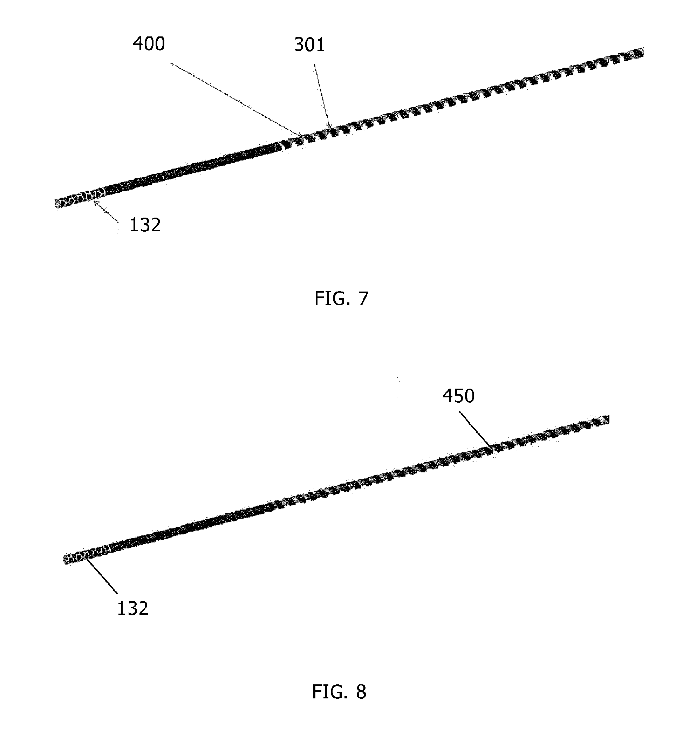

Figure 7 shows the assembly of the thin film 301 on the core 410. The distal array of electrodes 132 is mounted over the distal end of the core 410 and e.g. wrapped and glued around the core 410. The thin film 301 is then wound and bonded on the core 410, from the array of electrodes 132 over the spring structure 420 till the proximal end.

As shown in Figure 8, the depth tubing 450 is then inserted inside the core tube 410 and its distal end is attached (for instance via adhesive bonding) to the distal end of the core 410. The depth tubing 450 is made of biocompatible polymer, preferably with excellent lubricity properties such as PEEK or polyurethane.

Advantageously, the depth tubing 450 had some markings/graduations 470 along its length to indicate the depth of implantation as well as the azimuthal orientation of the electrodes (see Figure 9) and opacity markers for X-Ray visibility. Generally, the fiducial marker 470 may be e.g. a marker which is directly visible and/or visible with imaging technologies such as X-Ray, computer tomography (CT), magnetic resonance imaging (MRI) or the like.

Figure 10 shows a schematical drawing of the length adjustment mechanism according to Figure 8 covered by a protective coating.

The thin protective coating is placed over the thin film, except for the part of the thin film 301 which is located on the spring structure 420 of the core 410 and on the proximal end of the core tube 410. Any coating techniques such as dip-coating, knife-coating, spray-coating, selective coating etc. can be used to coat the thin film 301. The protective coating is made of biocompatible polymer such as silicone or polyurethane and may be e.g. about 30 microns thick.

The distal 60mm of the lead 300 has only the lead coating over the thin film 301. The remainder of the lead length may have a thin overlay 460 to provide a smooth and lubricious surface for the lead 300, as shown in Figure 10. The overlay 460 is made of biocompatible polymer such as polyurethane, PEEK etc. The distal end of the overlay 460 has a sealing lip to prevent fluid ingress in between the overlay 460 and the thin film 301. The proximal end of the overlay 460 is bonded to the proximal end of the core 410 e.g. via adhesive bonding.

## Claims

1. A lead (300), especially a lead (300) for neural applications, preferably a lead (300) for a neurostimulation and/or neurorecording system, wherein the lead (300) comprises at least one length adjustment mechanism (400), wherein the length adjustment mechanism (400) is configured such that the length of the lead (300) is adjustable.

2. The lead (300) according to claim 1,
**characterized in that**
the length adjustment mechanism (400) comprises at least one tubular section (410) and/or at least one rod-shaped section and/or that the length adjustment mechanism (400) comprises or is a tube and/or a rod.

3. The lead (300) according to claim 1 or 2,
**characterized in that**
the length adjustment mechanism comprises at least one partially spirally wound section (420), wherein the at least one partially spirally wound section is configured such that the length of the lead (300) is adjustable, especially that the at least one partially spirally wound section (420) is configured such that the length of the lead (300) is adjustable by extending or compressing the one partially spirally wound section (420).

4. The lead (300) according to claim 2,
**characterized in that**
the spirally wound section (420) forms a spring structure and/or that spirally wound section (420) is connected to the tubular section.

5. The lead (300) according to one of claims 1 to 4,
**characterized in that**
the lead (300) comprises a thin film (301) and that the thin film (301) is at least partially attached to the length adjustment mechanism (400).

6. The lead (300) according to one of claims 1 to 5,
**characterized in that**
the lead (300) comprises at least one reinforcement means (450), which is configured and/or arranged such that the overall structure of the lead is at least partially stabilized and/or reinforced, wherein especially the reinforcement means (450) is at least partially made of a biocompatible and/or biostable polymeric material, wherein further especially the polymeric material is PEEK and/or polyurethane or the like.

7. The lead (300) according to claim 6,
**characterized in that**
the reinforcement means (450) is connectable and/or insertable and/or attachable with or into the length adjustment mechanism (400) and/or that the length adjustment mechanism (400) is connectable and/or insertable and/or attachable with or into the reinforcement means (450).

8. The lead (300) according to claim 6,
**characterized in that**
the reinforcement means (450) and/or the length adjustment mechanism (400) comprises a length indication means and/or an azimuthal orientation means, exemplarily at least one fiducial marker (470), and/or that the reinforcement means (450) and/or the length adjustment mechanism (400) is configured such that the relative position to at least one element of the length adjustment mechanism (400) is indicated.

9. The lead (300) according to one of claims 1 to 9,
**characterized in that**
the length adjustment mechanism (400) is at least partially directly and/or indirectly covered by a protective coating (460), wherein especially the protective coating (460) is a biocompatible and/or biostable protective coating (460).

10. The lead (300) according to one of claims 1 to 9,
**characterized in that**
the lead (300) comprises a locking mechanism which is configured such that the position of the at least one partially spirally wound section and at least one fixation means is lockable such that the length of the lead (300) is fixable after the length adjustment.

11. The lead (300) according to one of claims 1 to 10,
**characterized in that**
the least length adjustment mechanism (400) is at least partially made of a biocompatible and/or biostable material, wherein the biocompatible and/or biostable material is exemplarily a biocompatible and/or biostable polymer such as silicone, PEEK, polyurethane or the like and/or a biocompatible and/or biostable metal and/or metal alloy, and where further exemplarily the at least one tubular section (410) and/or at least one rod-shaped section and/or the tube and/or the rod and/or the at least one partially spirally wound section (420) is at least partially made of a biocompatible and/or biostable material, wherein the biocompatible and/or biostable material is exemplarily a biocompatible and/or biostable polymer such as silicone, PEEK, polyurethane or the like and/or a biocompatible and/or biostable metal and/or metal alloy.

12. A neurostimulation and/or neurorecording system (100), especially a deep brain stimulation (DBS) system (100), comprising at least one lead (300) according to any of the preceding claims.

13. A thin film (301) comprising the thin film features according to any of the claims 1 to 11.

14. A fixation means comprising the fixation means features according to any of the claims 1 to 11.

15. A locking mechanism comprising the locking mechanism features according to any of the claims 1 to 11, wherein especially the locking mechanism is a snap-fit locking mechanism and/or wherein especially the locking mechanism comprises a snap-fit locking mechanism.
